Europäisches Patentamt

(19) European Patent Office

Office européen des brevets

(11) Publication number: **0 216 972**
**B1**

# EUROPEAN PATENT SPECIFICATION

(12)

(45) Date of publication of the patent specification:
07.02.90

(51) Int. Cl.⁴: **C07C 1/04**, **B01J 23/00**,
**B01J 23/74**, **B01J 23/78**

(21) Application number: 85306917.7

(22) Date of filing: 27.09.85

(54) Fischer-Tropsch process with cobalt-promoted catalysts.

(43) Date of publication of application:
08.04.87 Bulletin 87/15

(45) Publication of the grant of the patent:
07.02.90 Bulletin 90/6

(84) Designated Contracting States:
**DE GB NL**

(56) References cited:
**DE-A- 2 919 921**
**US-A- 4 228 138**
**US-A- 4 518 707**

(73) Proprietor: **Exxon Research and Engineering Company,**
**P.O.Box 390 180 Park Avenue, Florham Park New**
**Jersey 07932(US)**

(72) Inventor: **Flato, Rocco Anthony, 275 Country Club Lane,**
**Scotch Plains New Jersey 07076(US)**
Inventor: **Montagna, Angelo Anthony, 520 Shady Lane**
**Parkway, Baton Rouge Louisiana 70810(US)**
Inventor: **Soled, Stuart Leon, RD 1, P.O. Box 117 Cooks**
**Cross Lane, Pittstown New Jersey 08867(US)**

(74) Representative: **Somers, Harold Arnold et al, ESSO**
**Engineering (Europe) Ltd. Patents & Licences Apex**
**Tower High Street, New Malden Surrey KT3 4DJ(GB)**

## Description

### FIELD OF THE INVENTION

This invention relates to a Fischer-Tropsch process for producing high amounts of $C_2$ to $C_{20}$ olefins, particularly those in the $C_2$–$C_4$ range, using as a catalyst, an unsupported iron-cobalt single phase spinel, in which the atomic ratio of Fe:Co is 7:1 or above.

### DISCLOSURES IN THE ART

Fischer-Tropsch processes have long been known to produce gaseous and liquid hydrocarbons containing olefins. Because of the importance of these olefins, particularly as feedstocks for the chemical industry, modifications of the Fischer-Tropsch process are constantly being pursued toward the goals of maximizing olefin selectivity with the particular objective of maintaining high catalyst activity and stability under the reaction conditions. The main thrust of the efforts in this area has been in the area of catalyst formulation.

Coprecipitated iron-based catalysts, including those containing cobalt, are known for producing olefins. High levels of cobalt in an iron-cobalt alloy are known to produce enhanced selectivity to olefinic products, as described in Stud. Surf. Sci. Catal. 7, Pt/A, pp. 432 (1981).

Other disclosures in the art directed to coprecipitated iron-cobalt catalysts and/or alloys include: US-A 2 850 515, US-A 2 686 195, US-A 2 662 090, and US-A 2 735 862; AICHE 1981 Summer Nat'l Meeting Preprint No. 408, "The Synthesis of Light Hydrocarbons from CO and $H_2$ Mixtures over Selected Metal Catalysts" ACS 173rd Symposium, Fuel Division, New Orleans, March 1977; J. Catalysis 1981, No. 72(1), pp. 37–50; Adv. Chem. Ser. 1981, 194, 573–88; Physics Reports (Section C of Physics Letters) 12 No. 5 (1974) pp. 335–374; UK Patent Application No. 2 050 859A; J. Catalysis 72, 95–110 (1981); Gmelins Handbuch der Anorganische Chemie 8, Auflage (1959), pp. 59; Hydrocarbon Processing, May 1983, pp. 88–96; and Chem. Ing. Tech. 49 (1977) No. 6, pp. 463–468.

There is further disclosed a method for producing high surface area metal oxides in the French article, "C. R. Acad. Sc. Paris", p. 268 (28 May 1969) by P. Courte and B. Delmon. The article describes a process for producing high surface area metal oxides by evaporating to dryness aqueous solutions of the corresponding glycolic acid, lactic acid, malic or tartaric acid metal salts. One oxide that was prepared by their described method was $CoFe_2O_4$.

However, the above references do not describe or suggest the use of single phase iron-cobalt spinels having an Fe:Co atomic ratio of 7:1 or above or suggest their applicability in conducting or carrying out Fischer-Tropsch processes for synthesizing olefins.

What is particularly desired in fixed bed Fischer-Tropsch processes are new catalysts for selectively producing high levels of olefins and low levels of methane under the desirable combined conditions of high catalyst activity and stability.

US-A 4 518 707 describes and claims, in one aspect, a composition of matter comprising an unsupported, Group IA or IIA metal salt promoted iron-cobalt spinel, or mixture thereof, said spinel exhibiting a single phase powder X-ray diffraction pattern substantially isostructural with $Fe_3O_4$, and possessing a BET surface area greater than 5 m2/g and an iron-cobalt atomic ratio of 4 to 1 or above. The BET surface area is preferably at least 50 m2/g, more preferably in the range 100 to 300 m2/g.

US-A 4 518 707 also describes and claims a composition of matter comprising a reduced and carbided iron-cobalt alloy, said composition being substantially isostructural with chi-$Fe_5C_2$, as determined by X-ray diffractometry and possessing a BET surface area of greater than 5 m2/g, said composition produced by contacting, with a carbiding atmosphere for a time sufficient to produce said composition, said iron-cobalt alloy being isostructural with metallic alpha iron as determined by powder X-ray diffractometry, and possessing a BET surface area of greater than 5 m2/g, wherein said alloy was produced by contacting, with a reducing atmosphere, an unsupported Group IA or IIA metal salt promoted iron-cobalt spinel, or mixture thereof, said spinel exhibiting a single phase powder X-ray diffraction pattern substantially isostructural with $Fe_3O_4$, and possessing a BET surface area greater than 5 m2/g and an iron-cobalt atomic ratio of 4 to 1 or above.

The composition is said to be useful as a catalyst for use in Fischer-Tropsch reactions for the production of $C_2$ to $C_{20}$ alpha-olefins, particularly in slurry processes.

US-A 4 518 707 further describes and claims a process for producing the composition comprising the steps of (a) evaporating a liquid solution comprising a mixture of iron and cobalt salts of at least one alpha-hydroxy aliphatic carboxylic acid, wherein the molar ratio of total moles of said acid to total moles of said iron and cobalt, taken as free metals, is about 1:1, or above, and wherein the atomic ratio of iron:cobalt, taken as the free metals in said mixture, is greater than 2 to 1, yielding an amorphous residue; (b) calcining said residue at elevated temperature for a time sufficient to yield an iron-cobalt spinel, exhibiting at least one spinel phase, isostructural with $Fe_3O_4$, as determined by powder X-ray diffractometry; and (c) impregnating the composition of (b) with an aqueous solution of a Group IA or Group IIA metal salt, followed by drying the resulting impregnate.

US-A 4 518 707 also describes and claims a process for preparing an iron-cobalt alloy, being isostructural with metallic alpha iron as determined by powder X-ray diffractometry, and possessing a BET surface area of greater than 5 $m^2/g$, which comprises contacting an unsupported, Group IA or Group IIA metal salt promoted iron-cobalt spinel, or mixture thereof, said spinel exhibiting a single spinel phase isostructural with $Fe_3O_4$, and having a BET surface area greater than 5 $m^2/g$ and an iron-cobalt atomic ratio of 4:1 or above, with a reducing atmosphere under conditions of elevated temperature, pressure, and space velocity, for a time sufficient to substantially reduce the metal oxides of the spinel.

The present invention provides a process for synthesizing a hydrocarbon mixture containing $C_2$ to $C_6$ olefins from a feed gas containing CO and $H_2$ comprising passing the feed gas in contact with a carbided and reduced, unsupported iron-cobalt single phase spinel catalyst, said spinel having the initial empirical formula:

$Fe_xCo_yO_4$

wherein x and y are integer or decimal values, other than zero, with the provisos that the sum of x+y is 3 and the ratio of x/y is 7:1 or above, said spinel exhibiting a single phase X-ray diffraction pattern substantially isostructural with that of $Fe_3O_4$ at a temperature in the range of from 200 to 300°C, a (gauge) pressure in the range of from 50 to 1000 psig (344.8 to 6895 kPa) and for a sufficient time to form a hy-drocarbon mixture containing $C_2$ to $C_6$ olefins, characterized in that the said spinel has a measured B.E.T. surface area in the range of from 0.1 to 5 $m^2/g$. The catalyst preferably comprises a promoter agent present in an amount of from 0.1 to 10 gram-atom percent of the total gram-atoms of metal content. The promoter agent may be selected from bicarbonates, carbonates, organic acid salts, inorganic acid salts, nitrates, sulfates, halides and hydroxides of Group IA and IIA metals (e.g. potassium carbonate).

It has been found that an unsupported iron-cobalt single phase spinel which is isostructural with $Fe_3O_4$ as determined by X-ray diffractometry and possesses an initial BET surface area of from 0.1 to 5 $m^2/g$ and greater and an iron:cobalt atomic ratio of 7:1 or greater provides desirable catalyst properties in Fischer-Tropsch processes for synthesizing a hydrocarbon mixture containing $C_2$–$C_6$ olefins from a feed gas containing CO and $H_2$.

The spinels used in the process of the invention can be conveniently prepared in a high temperature solid state sintering reaction in a temperature range of from 600 to 1100°C between stoichiometric amounts of mixtures of the component metal oxides and/or metals, in an inert or vacuum atmosphere. The spinels prepared in this manner can then be treated, if desired, with promoter agents, such as Group IA or IIA metal salts, and particularly potassium carbonate. The resulting combined iron and co-balt/potassium atomic ratio is desirably in the range of from 20:1 to 200:1. The promoted catalyst is then reduced in a hydrogen-containing gas and carbided before use in the Fischer-Tropsch process.

The iron-cobalt spinels used in the process of the invention are, as already noted, compositions of matter which are isostructural with $Fe_3O_4$, as determined by x-ray diffractometry using copper K alpha radiation and exhibit a single spinel phase. By the term "spinel" is meant a crystal structure whose gener-al stoichiometry corresponds to $AB_2O_4$, where A and B can be the same or different cations. Included within this definition is the commonly found spinel $MgAl_2O_4$. A and B can have the following cationic charge combinations: A=+2, B=+3, A=+4, B=+2, or A=+6, B=+1. Spinels are arranged of an approximately cubic close-packed arrangement of oxygen atoms with 1/8th of the available tetrahedral interstices and 1/2 of the octahedral interstices filled, and can exhibit hundreds of different phases. Further description of the spinel structure can be found in "Structural Inorganic Chemistry" by A.F. Wells, Third Edition, Oxford Press, and the article "Crystal Chemistry and Some Magnetic Properties of Mixed Metal Oxides With the Spinel Structure" by G. Blasse, Phillips Research Review Supplement, Volume 3, pp. 1–30 (1964). By the term "isostructural" is meant crystallizing in the same general structure type in that the arrangement of the atoms remains very similar with only minor changes in unit cell constants, bond energies and angles. By the term "single phase spinel", as used herein, is meant one structural and compositional formula, corresponding to a single spinel material into which all of the metal components are incorporated, and exhibiting one characteristic X-ray diffraction pattern.

The subject iron-cobalt spinel possesses a BET surface area of from 0.1 $m^2/g$ to 5 $m^2/g$, as determined by the well-known nitrogen gas BET surface area measurement technique as described in the reference JACS 60, p. 309 (1938) by S. Brunauer, P.H. Emmett, and E. Teller. Generally, the spinel prepared by the above-described sintering of component oxides has a surface area of about 0.1 to 1 $m^2/g$. This range of surface area generally corresponds to a particle size range of about 1 to 10 microns (μm).

The iron to cobalt atomic ratio of the metals in the spinel is 7:1 or above and is preferably in the range of from 7:1 to 35:1 and particularly preferred in the range of 19 to 20:1.

The spinel can be represented by the formula: $Fe_xCo_yO_4$, wherein x and y are decimal or integer values, other than zero, and wherein the sum of x plus y is 3, and the ratio of x to y is 7:1 or above and preferably being from 7:1 to 35:1. Particularly preferred is where the iron to cobalt atomic ratio is about 19 to 20:1.

Representative examples of the various spinels corresponding to the formula are $Fe_{2.85}Co_{0.15}O_4$, $Fe_{2.625}Co_{0.375}O_4$ and $Fe_{2.97}Co_{0.03}O_4$.

Physical properties in general of these subject spinels are similar to those of magnetite, $Fe_3O_4$, and include: melting point of above 1400°C, and color of brownish to blackish.

The iron-cobalt spinels are used in unsupported form in $H_2/CO$ hydrocarbon synthesis.

A promoter agent can also be used in the composition and can be used to particularly promote olefin formation in the process. Representative examples of classes of suitable promoter agents include alkali metal and alkaline earth metal salts including carbonates, bicarbonates, organic acid salts, i.e., acetates, inorganic acid salts, i.e. nitrates, halides, sulfates, and hydroxide salts of Group IA and IIA metals including lithium, sodium, potassium, cesium, rubidium, barium, strontium and magnesium. Preferably, the promoter agent is deposited or impregnated substantially on the surface of said spinel composition.

Representative examples of specific promoter agents are potassium carbonate, potassium sulfate, potassium bicarbonate, cesium chloride, rubidium nitrate, lithium acetate and potassium hydroxide. Preferred are the Group IA compounds and a particularly preferred promoter agent is potassium carbonate. The promoter, if used, is generally present in about a range of from 0.1 to 10 gram-atom % as the metal ion of the total combined metal gram-atoms present. A preferred level of promoter agent is in the range of from 1 to 2 gram-atom % of the total combined metal gram-atoms present. In the empirical formulas used herein, the amount of the promoter agent, e.g., potassium, is expressed in terms of gram atom percent based on the total gram-atoms of metals used. Thus, "1 gram-atom of potassium" signifies the presence of 1 gram-atom of potassium per 100 total gram atoms of combined gram atoms of Fe and Co. Thus, the symbol "1% K" as used herein indicates 1 gram-atom percent potassium based on each 100 gram atoms of the total combined gram atoms of iron and cobalt present.

A particularly preferred spinel composition of the subject invention is $Fe_{2.85}Co_{0.15}O_4/1\%K$ (potassium taken as the carbonate).

The catalyst spinel in the subject process may also be used in conjunction and admixture with a diluent material; one which aids in heat transfer and removal from the catalyst bed. Suitable materials include powdered quartz, silicon carbide, powdered borosilicate glass, $SiO_2$, porous silica, kieselguhr, zeolites, talc, clays, Group II to VII metal oxides and rare earth oxides including $TiO_2$, $SiO_2$, $Al_2O_3$, MgO, $La_2O_3$, $CeO_2$, $Cr_2O_3$ and $MnO_2$. Preferred is powdered quartz.

The diluent, if used, is generally used in a 1:4 to 9:1 diluent/spinel catalyst composition weight ratio. Preferred is a 1:1 weight ratio.

The utility of these spinels is their ability upon subsequent reduction and carbiding to form active catalysts in a Fischer-Tropsch process for making $C_2$–$C_{20}$ olefins from CO/hydrogen.

The reduced and carbided forms of the above-described spinel are also subjects of this invention.

As hereinabove described, a low surface area spinel, i.e., up to about 5 m²/g, can be prepared by a solid state high temperature reaction between (1) the component oxides, i.e. $Fe_3O_4$ and $Co_3O_4$, or (2) a mixture of iron metal, cobalt oxide and iron oxide, i.e. Fe metal, $Co_3O_4$ and $Fe_2O_3$, or (3) a mixture of cobalt metal, iron oxides and cobalt oxide, i.e. Co, $Fe_3O_4$, $Fe_2O_3$ and $Co_3O_4$ or (4) a mixture of iron and cobalt metals, iron oxide and cobalt oxide, i.e. Fe, Co, $Fe_2O_3$ and $Co_3O_4$, in the correct stoichiometric metals and oxygen ratio to result in the empirical formula for the composition as given above. Preferred is indicated reaction (1) between iron oxide and cobalt oxide. The reaction is conducted at temperatures in the range of from 600° to 1100°C and preferably from 800 to 900°C, in an inert gas, oxygen-free atmosphere, or vacuum environment. Examples of useful inert gases are helium, nitrogen and argon. The solid state high temperature reaction "sintering" should be performed on thoroughly mixed samples of the metal oxides and/or metal and metal oxide mixtures. A method of forming the mixture is by intimate grinding and shaking. The sintering reaction should be conducted until a powder X-ray diffraction pattern indicates a single spinel phase is formed, being isostructural with $Fe_3O_4$, which generally requires an 8 to 24 hour period and preferably a 12 to 18 hour period. Generally, at the end of each reaction period the material is thoroughly ground and mixed and then resubjected to the high temperature conditions for an additional 1 to 5 cycles or until powder x-ray diffraction reveals the presence of a single spinel phase.

Prior to the hydrocarbon synthesis run, the iron-cobalt spinel is reduced in a reducing atmosphere at elevated temperature, generally in a temperature range of from 200 to 500°C and preferably from 300 to 450°C. The reduction can be carried out with various reducing gases including hydrogen, CO, and mixtures thereof, and the llike. Preferably, hydrogen gas, either by itself or in an inert carrier medium such as helium, neon, argon, or nitrogen, is preferably used. The (gauge) pressure of the reducing gas in this procedure may be in the range of from 1.5 to 1000 psig (10 to 6950 kPa) and preferably in the range of 15 to 150 psig (103 to 1034 kPa). The reducing gas feed rate may be in the range of from 1–10,000 V/V/hr and preferably in the range of from 10–1000 V/V/hr. The reduction is carried out until the resulting Fe–Co alloy is substantially reduced and exhibits a powder X-ray diffraction pattern isostructural with alpha iron. This reduction usually requires from 2 to 20 hours.

In the reduction treatment of the low surface area spinel, the resulting reduced spinel may have a BET surface area of up to 3 m²/g and is useful in forming a carbided iron–cobalt catalyst for the Fischer-Tropsch process of the invention for making $C_2$ to $C_6$ olefins as described herein.

The iron-cobalt alloy can be prepared _ex situ_ in a tube reactor or _in situ_ in a Fischer-Tropsch slurry process. The _in situ_ preparation is conducted in the slurry apparatus when the above-described spinel is reduced while suspended in the slurry liquid, in a reducing atmosphere being preferably a hydrogen atmosphere at elevated temperature being about 240°C, or above, preferably at from 240–300°C, at a space velocity, pressure, and hydrogen concentration sufficient to cause substantial reduction of the spinel to the alloy. Substantial reduction is complete when the X-ray diffraction pattern shows a pattern substantially isostructural with alpha-iron.

The iron-cobalt catalyst which is believed to be the primary active catalyst in the process can be produced by carbiding the reduced iron-cobalt spinel, described hereinabove, generally having an X-ray diffraction pattern isostructural with chi $Fe_5C_2$ (Hagg carbide), by heating at elevated temperature in a suitable carbiding atmosphere, containing CO, $H_2/CO$, and mixtures thereof. The spinel can also be reduced and carbided, concurrently, by contact with a $CO/H_2$ atmosphere under the hydrocarbon synthesis conditions described below.

Carbiding atmospheres which can be used include CO/hydrogen, aliphatic hydrocarbons, acetylene and aromatic hydrocarbons. A preferred carbiding atmosphere is CO/hydrogen. When using a CO/hydrogen carbiding atmosphere, mixtures of CO/hydrogen can be used in a 10:1 to 1:10 molar volume ratio. A preferred ratio used for carbiding purposes is 1:1 molar ratio.

The carbiding step is generally conducted at a temperature of from 300 to 450°C and preferably from 350 to 400°C. The (gauge) pressure is generally about 0.30 psig (2.1 kPa), and a space velocity of about 200–1000 V/V/hr are chosen in order to completely carbide the reduced iron-cobalt spinel which can be subjected to X-ray diffractometry to determine when the material becomes isomorphous with chi-$Fe_5C_2$. At carbiding temperatures above about 450°C, the resulting Hagg type carbide, $Fe_{5-(5/3)y}Co_{(5/3)y}C_2$, becomes unstable and can rearrange crystallographically to the corresponding cementite type structure, $Fe_{3-y}Co_yC$. Also, in the ex situ carbiding step, a significant amount of carbon is also formed on the surface of the catalyst which tends to increase the surface area of the reduced, carbided catalyst.

A preferred method of carbiding the alloy is in situ in the slurry liquid to be used in a slurry Fischer-Tropsch process. A particularly preferred method is where the spinel is treated with a mixture of CO/hydrogen and reduced and carbided in situ in one step prior to hydrocarbon synthesis. The pressure is generally about 1 atmosphere, and a space velocity of about 20–20,000 V/V/hr is chosen in order to completely carbide the starting iron-cobalt oxide which can be determined by X-ray diffractometry when the material becomes isostructural with Haag carbide, $Fe_5C_2$. The Haag-type Fe–Co carbides produced in this process are of the general formula: $Fe_{5-(5/3)y}Co_{(5/3)y}C_2$, and also include surface carbon produced during the carbiding process. Carbiding temperatures above 500°C and preferably from 500–700°C, lead to formation of a mixed Fe–Co carbide of the general formula $Fe_{3-y}Co_yC$, which is generally formed under ex situ procedures which allow the use of higher temperatures than possible in the in situ slurry process.

The above-described reduced spinel and carbided spinel, when prepared ex situ are generally pyrophoric and inconvenient to handle. In that case, the material is generally passivated by contact with 1 volume oxygen in 100 volumes of helium for a sufficient time to reduce or eliminate the pyrophoric nature. Generally, the oxygen used in the passivating process is used in an inert gas stream carrier such as helium for a sufficient time to cause passivation. Generally, this is conducted at room temperature and atmospheric pressure and space velocity which are convenient and easy to control and to result in an efficient process needed for complete passivation.

The Fischer-Tropsch process of the invention utilizing the catalysts described herein may be operated as a fixed bed process or as a slurry-type process wherein the catalyst is suspended in a liquid hydrocarbon and the CO/hydrogen mixture forced through the catalyst slurry allowing good contact between the CO/hydrogen and the catalyst to initiate and maintain the hydrocarbon synthesis process.

Advantages of a slurry process over that of a fixed bed process are that there is better control of the exothermic heat produced in the Fischer-Tropsch process during the reaction and that better control over catalyst activity maintenance by allowing continuous recycle, recovery, and rejuvenation procedures to be implemented. The slurry process can be operated in a batch or in a continuous cycle, and in the continuous cycle, the entire slurry can be circulated in the system allowing for better control of the primary products residence time in the reaction zone.

The slurry liquid used in the process is a liquid at the reaction temperature, must be chemically inert under the reaction conditions and must be a relatively good solvent for CO/hydrogen and possess good slurrying and dispersing properties for the finely divided catalyst. Representative classes of organic liquids which can be utilized are high boiling paraffins, aromatic hydrocarbons, ethers, amines, or mixtures thereof. The high boiling paraffins include $C_{10}$–$C_{50}$ linear or branched paraffinic hydrocarbons; the aromatic hydrocarbons include $C_7$–$C_{20}$ single ring and multi- and fused ring aromatic hydrocarbons; the ethers include aromatic ethers and substituted aromatic ethers where the ether oxygen is sterically hindered from being hydrogenated; the amines include long chain amines which can be primary, secondary, and tertiary amines wherein primary amines preferably contain at least a $C_{12}$ alkyl group in length, secondary amines preferably contain at least two alkyl groups being $C_9$ of greater in length, and tertiary amines preferably contain at least three alkyl groups being $C_6$ or higher in length. The slurry liquid can contain N and O in the molecular structure but not S, P, As or Sb, since these are poisons in the slurry process. Representative examples of specific liquid slurry solvents useful are dodecane, tetradecane, hexadecane, octadecane, cosane, tetracosane, octacosane, triacontane, dotriacontane, hexatriacon- tane, tetracontane, tetratetracontane, toluene, o-, m-, and p-xylene, mesitylene, $C_1$–$C_{13}$ mono- and multi-alkyl substituted benzenes, dodecylbenzene, naphthalene, anthracene, biphenyl, diphenylether, dodecylamine, dinonylamine and trioctylamine. A preferred liquid hydrocarbon slurry solvent is octacosane.

The amount of catalyst used in the liquid hydrocarbon slurry solvent is generally from 10 to 60 g. of dry catalyst per 500 g. slurry liquid. Preferably, from about 30 to 50 g. dry catalyst per 500 g. slurry liquid slurry is utilized, being in a respective 5:1 to 10:1 weight ratio.

The slurry system, comprised of the slurry liquid and finally divided catalyst, is generally stirred to promote good dispersion during the pretreatment process to avoid catalyst settling and to eliminate mass transport limitations between the gas and liquid phases. In a typical laboratory unit the rate of stirring is generally carried out in the range of 600 to 1200 rpm and preferably 1000 to 1200 rpm.

Prior to the CO/hydrogen hydrocarbon synthesis run, the reduced and carbided iron-cobalt catalyst is generally conditioned in the slurry by purging with nitrogen to remove reactive oxygen-containing gases and then the temperature is increased while stirring to the reaction temperature range. Then the system is generally subjected to a hydrogen treatment for a sufficient time to insure complete removal of any surface metal oxide present which would interfere in hydrocarbon synthesis. The pressure and space velocity during the inert gas-hydrogen conditioning step are not critical and can be utilized in the range which is actually used during actual hydrocarbon synthesis.

Following the hydrogen reduction step, the CO/hydrogen feedstream is introduced into the slurry catalyst chamber and the pressure, space velocity, temperature, and hydrogen/CO molar ratio is then adjusted, as desired, for hydrocarbon synthesis conditions.

In the slurry process, the hydrogen and CO are used in a molar ratio in the gaseous feedstream in a 10:1 to 1:10 molar ratio, preferably 3:1 to 0.5:1, and particularly preferred 1:1 to 2:1 molar ratio.

The temperature in the slurry process is generally in the range of from 200 to 300°C, preferably being 230 to 270°C, and particularly preferred of from 240–260°C. Higher temperature ranges can also be used but tend to lead to lighter products and more methane, lower temperature ranges can also be used but tend to lead to lower activity and wax formation.

The (gauge) pressure useful in the slurry process is generally conducted in the range of from 50 to 400 psig (344.8 to 2758 kPa) and preferably from 70 to 255 psig (482.7 to 1551 kPa). Higher pressures can also be used but tend to lead to waxy materials particularly in combination with lower temperature.

The space velocity, expressed as standard hourly space velocity (SHSV), used in the slurry process is generally 100 to 4,000 volumes of gaseous feedstream/per volume of dry catalyst in the slurry/per hour and is preferably in the range of 400 to 1,200 V/V/hr, and particularly preferred of 800–1,200 V/V/hr. Higher space velocities can also be used but tend to lead to lower % CO conversions, and lower space velocities can also be used but tend to lead to more paraffinic products.

Generally, after the pretreatment the CO/hydrogen feedstream is introduced to initiate and maintain hydrocarbon synthesis. By the use of the above-described catalysts in the system, the activity maintenance is very good and on a laboratory scale, e.g., 500 $cm^3$ of slurry containing 50 g. of catalyst described herein, 30 days of continuous run have been observed without significant decline in percent CO conversion activity while maintaining good olefin synthesis activity.

The percent CO conversion obtainable in the subject process, while providing substantial quantities of olefins, ranges from 30 to 80 percent or higher.

"Total hydrocarbons" produced in the process is related to the selectivity of percent CO conversion to hydrocarbons being hydrocarbons from $C_1$ to about $C_{40}$ inclusive and is generally 0 to 75 percent of the total CO converted, the remainder being converted to $CO_2$.

In the process of the invention, the percent $C_2$–$C_6$ hydrocarbons of the total hydrocarbons produced including methane and above is from 10 to 30 wt.%. The percent of $C_2$–$C_6$ olefins produced, of the $C_2$–$C_6$ total hydrocarbons produced is 80 to 90 wt.%. The olefins produced in the process are substantially alpha olefins.

The selectivity to methane based on the amount of CO conversion is from 1 to 10 weight percent of total hydrocarbons produced. Preferably about 5 percent, and lower, methane is produced in the process.

Preferably, the reaction process variables are adjusted to minimize $CO_2$ production, minimize methane production, maximize percent CO conversion, and maximize percent $C_2$–$C_{20}$ and particularly $C_2$–$C_4$ olefin selectivity, while achieving activity maintenance in the catalyst system.

Generally, this format can be derived in a preferred mode of operating the process where the slurry liquid used is hexadecane, the catalyst used is reduced, carbided $Fe_{2.85}Co_{0.15}O_4$/1 % K as $K_2Co_3$, the catalyst liquid weight ratio is 40/500, the system is stirred at 600–1200 rpm, and the pretreatment procedure is conducted _in situ_ in a single step using 9:1 $H_2/N_2$, at 220°C, atmospheric pressure, 1200 V/V/hr space velocity for a period of up to 5 hours, and the olefins synthesis process conducted at a hydrogen:CO molar ratio of 1:1, a temperature of about 245°C, a (gauge) pressure of 7–150 psig (48.3 to 1034.3 kPa), and space velocity 1000–1200 V/V/hr. By carrying out the above process in the stated variable ranges efficient activity maintenance and production of $C_2$–$C_{20}$ and particularly $C_2$–$C_6$ olefins can be achieved.

The effluent gases in the process exiting from the reactor may be recycled, if desired, to the reactor for further CO hydrocarbon synthesis.

As hereinbefore mentioned, the subject process may be carried out as a fixed bed process utilizing the claimed catalysts described herein.

Prior to the CO/hydrogen hydrocarbon synthesis fixed bed run, the iron-cobalt spinel is generally conditioned in the apparatus by purging with nitrogen to remove reactive gases and then the temperature is increased to the reaction temperature range. Then the system is generally subjected to the above-described hydrogen treatment for a sufficient time to insure complete reduction of metal oxides. However, the pressure, space velocity, and temperature during this reduction step are not critical and can be utilized in the range which is actually used during actual hydrocarbon synthesis.

Following the reduction step, the CO/hydrogen feedstream is introduced into the fixed bed apparatus catalyst chamber and the pressure, space velocity, temperature, and hydrogen/CO molar ratio are then adjusted as desired, for hydrocarbon synthesis conditions. Optionally, the reduction/carbiding can be carried out concurrently by contact with the $CO/H_2$ mixture at elevated temperature.

In the fixed bed process, the hydrogen and CO are used in a molar ratio in the gaseous feedstream of a 10:1 to 1:10, preferably a 3:1 to 0.5:1 molar $H_2/CO$ ratio and more preferably 1:1 to 2:1 molar ratio. Higher and lower molar ratios may also be used.

The temperature in the fixed bed process is generally in the region of from 200 to 300°C and preferably from 250 to 280°C. Higher temperatures, such as in the range of from 300–350°C, tend to promote higher % CO conversion, lighter products, more methane and more $CO_2$, formed from the water-gas shift reaction.

The (gauge) pressure useful in the fixed bed process is generally conducted in the range of from 50 to 1000 psig (344.8 to 6895 kPa) and preferably from 100 to 300 psig (689.5 to 2068.5 kPa). Higher and lower pressures can also be used.

The space velocity, used in the fixed bed process is expressed as "standard" hourly space velocity (SHSV) and is generally from 200 to 4000 volumes of gaseous feedstream/per volume of dry catalyst (excluding diluent)/per hour and is preferably in the range of from 400 to 1200 V/V/hr. Higher and lower space velocities can also be used where higher space velocities tend to lead to increased olefin contents but decreased % CO conversion.

The percent CO conversion obtainable in the subject fixed bed process while providing substantial quantities of $C_2-C_6$ olefins, ranges from 20 to 98% and preferably above about 30%. Higher and lower ratio percentages of CO conversion may also be utilized.

"Total hydrocarbons" produced in the fixed bed process is related to the selectivity of percent CO conversion to hydrocarbons, being hydrocarbons from $C_1$ to about $C_{40}$ and above inclusive, and is generally 0 to 50 percent, and higher, of the total CO converted and the remainder being substantially converted to $CO_2$.

The percent total $C_2-C_6$ hydrocarbons of the total hydrocarbons produced in the fixed bed process, including olefins and paraffins is generally from 20 to 80 wt.% and preferably from 50 to 80 wt.%. The percent of $C_2-C_6$ olefins produced of the $C_2-C_6$ total hydrocarbons produced is generally from 50 to 90 wt.% and preferably from 70 to 90 wt.% of the $C_2-C_6$ total hydrocarbons. The olefins produced in the process are substantially alpha olefins.

The selectivity to methane in the fixed bed process based on the amount of CO conversion is from 2 to 12 weight percent of total hydrocarbons produced. Preferably about 10 percent and less methane is produced in the process.

As discussed above, the percent selectivity to $CO_2$ formation in the process is in the range of 10 to 50 percent of CO converted, and generally 30 to 50 percent.

The reaction process variables in the fixed bed process are preferably adjusted to minimize $CO_2$ production, minimize methane production, maximize percent CO conversion, and maximize percent $C_2-C_6$ olefin selectivity, while achieving activity maintenance in the catalyst system.

The catalyst in the process may become contaminated with high molecular weight hydrocarbons on exposure to carbon monoxide hydrogenation reaction conditions. As a result of this, catalyst activity may be diminished. In the event that this is observed it may be possible to recover nearly full catalyst activity by exposing the catalyst to a solvent wash and/or hydrogen treatment at elevated temperatures. We have found that this procedure can in some cases restore the catalyst with its initial performance characteristics.

Generally, this format can be achieved in a preferred mode of operating the fixed bed process where the formula of the catalyst used is $Fe_{2.85}Co_{0.15}O_4/1\%$ K. The pretreatment procedure is conducted at 500°C in a 9:1 $H_2/N_2$ stream at 680 v/v/hr. under 100 psig (689.5 kPa gauge) for 5–7 hours, and the hydrocarbon synthesis run is conducted at the CO/hydrogen molar ratio is 1:1 to 2:1, the temperature is conducted in the range 230–270°C, at a (gauge) pressure of from 150–300 psig (1034.3 to 2068.5 kPa), and space velocity of from 1000–1800 v/v/hr (SHSV). By carrying out the above process in the stated variable ranges, efficient activity maintenance and production of $C_2-C_6$ olefins can be achieved.

The effluent gases in the process exiting from the reactor may be recycled if desired to the reactor for further CO/hydocarbon synthesis.

Methods for collecting the products in the process are known in the art and include distillation and fractional distillation. Methods for analyzing the product liquid hydrocarbons and gaseous streams are also known in the art and generally include gas chromatography, liquid chromatography and high pressure liquid chromatography.

Apparatus useful in the subject process can include any conventional fixed bed type reactor, being horizontal or vertical, moving bed, fluid bed, and the like or any conventional slurry-type reactor for carrying out the slurry-phase type of operation. The slurry-type reactors can be horizontal or vertical, stationary or cyclical. Other apparatus not specifically described herein will be obvious to one skilled in the art from a reading of this disclosure.

The following examples are illustrations of the best mode of carrying out the claimed invention as contemplated by us.

EXAMPLE 1

Solid solutions with the generic empirical formula: $Fe_{3-y}Co_yO_4/1\%K$ (1 gram-atom percent potassium as the carbonate) were prepared by the following procedure. Mixtures of $Fe_2O_3$, Fe metal and $Co_3O_4$ in the following molar ratios, $(4/3 - 4y/9) Fe_2O_3 + 1/3 (1-y/3) Fe + y/3 Co_3O_4$, where the value of y independently was: 0, 0.03; 0.150; 0.375; and 0.750, corresponding respectively to the following weights in grams of $Fe_2O_3$, Fe metal, and $Co_3O_4$; 21.080, 1.8400, 0.00; 22.750, 1.9891, 0.2594; 21.797, 1.9054, 1.2974; 20.0163, 1.7502, 3.2338; 11.381, 0.9590, 4.2904. The materials (reagent quality or better from Alfa Chemicals Co.) were well mixed, placed into a quartz tube, evacuated to $10^{-3}$ torr (0.133 kPa), sealed in the tube under vacuum and then heated to 800°C for 24 hours. The resulting solids were isolated after cooling and breaking the tube open, ground to a powder, and resubjected to the same high temperature sintering procedure, at 800° to 1000°C for an additional 24 hours. Powder X-ray diffraction analysis was then conducted to ensure that the sintered material was isostructural with pure standard sample of $Fe_3O_4$. The catalyst powder was then pelletized and sintered in a sealed tube as described above under vacuum at 1000°C for several hours. The sintered pellets were then crushed, sieved and the resulting pellets impregnated with aqueous potassium carbonate to achieve the desired potassium loading, being about 1 gram-atom percent potassium, and dried. The BET (nitrogen) surface areas measured were in the range of from 0.25 to 0.30 m²/g. The results are listed below in Table I.

TABLE I

| Composition | $Fe_{3-y}Co_yO_4/1\%$ K | |
|---|---|---|
| | y | BET (m²/g) |
| Control | 0.00 | 0.27 |
| A | 0.0275 | 0.30 |
| B | 0.150 | 0.29 |
| C | 0.375 | 0.25 |
| D | 0.750 | 0.28 |

The powder x-ray diffraction spectrum of each of the obtained Fe–Co spinels showed that they were a single phase and isostructural with $Fe_3O_4$. They differed from one another in slight shifts of the 2 theta reflection values without altering the overall profile.

EXAMPLE 2

Catalyst B, $Fe_{2.85}Co_{0.15}O_4/1\%$ K, where y = 0.15, was prepared by the procedure described in Example 1. X-ray diffraction analysis showed this material to be isostructural with $Fe_3O_4$, although there was a slight change in the unit cell constant where the unit cell constant is about 0.01 to 0.02 Å (0.1 to 0.2 nm) smaller than that of $Fe_3O_4$. The sintered material was found to have a low surface area, less than 5 m²/g. This material was crushed and sieved to 20–80 mesh before use in this example under F-T (Fischer-Tropsch) fixed bed reaction conditions. The reactor was charged with 8.8 cm³ of catalyst with a thermocouple placed at the center of the bed. The catalyst compositions of 20–80 mesh particle size, were pretreated with hydrogen gas in nitrogen (90% hydrogen/nitrogen) at 500°C, 100 sccm (680 v/v/hr.) of hydrogen gas at 100 psig (689.5 kPa gauge) for 5 to 7 hours in a fixed bed tubular vertical reactor constructed of 316 stainless steel, and being 0.51″ (12.95 mm) internal diameter and 15″ (38.1 cm) long. The runs were conducted using a 1:1 $H_2/CO$ mixture, at 570 v/v/hr., 300 psig (2068.5 kPa gauge), at the indicated temperatures, which are furnace temperatures in this and the remaining examples unless otherwise indicated as bed temperatures. In many of the cases, the bed temperature was 10–30°C higher than the indicated furnace temperature, due to primarily to the limited heat removal capabilities of the reactor system and the highly exothermic nature of the reaction. The overall collected products which were collected after catalyst pretreatment, and one hour on stream with $CO/H_2$, were analyzed by gas chromatography.

Representative results obtained with catalyst composition B, $Fe_{2.85}Co_{0.15}O_4/1\%K$, relative to the control (see Table I) are presented below in Table II.

**TABLE II**

| Catalyst | $Fe_3O_4/1\%K$[a] | $Fe_{2.85}Co_{0.15}O_4/1\%K$[b] |
|---|---|---|
| Temp. °C | 305 | 270 |
| % CO Conversion | 79 | 98 |
| % CO to $CO_2$ | 36 | 42 |
| % CO to $HC$[c] | 43 | 56 |
| Wt. % Selectivity | | |
| $CH_4$ | 8.5 | 9.1 |
| $C_2H_6$ | 2.1 | 4.3 |
| $C_2H_4$ | 6.5 | 9.8 |
| $C_3H_8$ | 1.4 | 1.9 |
| $C_3H_6$ | 10.6 | 20.3 |
| $C_4H_{10}$ | 1.7 | tr. |
| $C_4H_8$ | 9.5 | 9.3 |
| $C_5^+$ | 59.7 | 45.2 |

[a]Control.
[b]Composition B.
[c]Hydrocarbons.

As is seen from the data, Catalyst B, derived from the cobalt-containing spinel, exhibited greater activity at lower temperatures and higher $C_2$–$C_4$ olefin selectivity than the all iron control catalyst.

It should be noted that unless stated differently herein, the catalysts used in each of the following examples were in powder form of 20–80 mesh, used as is, or diluted with crushed quartz powder, totalling a catalyst volume of about 8–8.8 $cm^3$.

Further, the apparatus used was the same as described in this Example 2 and the pretreatment procedure was substantially the same as described in Example 2.

Values for selectivity weight percentages of product hydrocarbons are reported on a $CO_2$-free basis unless otherwise stated.

EXAMPLE 3

Four (4) $cm^3$ of Catalyst B, described above in Example 2, was mixed with 20–80 mesh solid quartz powder (crushed quartz tubes) in 4.0 $cm^3$ quantity, and the mixture was placed into the reactor described in Example 1, and pretreated by contacting with a 9:1 $H_2/N_2$ feedstream at 500°C, 750 v/v/hr., 100 psig (689.5 kPa gauge), for 5.5 hours.

The mixed diluted catalyst was then contacted with 1:1 $H_2/CO$ at 270°C, 300 psig (2068.5 kPa gauge), at 2000 v/v/hr. for 12 hours on stream. The product distribution was analyzed by gas chromatography, and the results are given below in Table III.

**TABLE III**

| Catalyst | 1:1 Catalyst B/quartz powder |
|---|---|
| % Conversion | 62 |
| % CO to $CO_2$ | 24 |
| % CO to H.C. | 38 |
| Wt. % Selectivity | |
| $CH_4$ | 9.2 |
| $C_2^o$–$C_5^o$ | 7.9 |
| $C_2^=$–$C_5^=$ | 48.2 |
| $C_6^+$ | 34.7 |

As is seen from the data, the catalyst derived from the iron-cobalt spinel provides good activity and high $C_2$–$C_5$ olefin selectivity with high $H_2/CO$ feed rates.

EXAMPLE 4

Catalyst B, in a 1:1 admixture with crushed quartz, as described in Example 3, was run under a different set of F–T synthesis conditions as described below.

Following substantially the same pretreatment, described in Example 3, about 8 cm³ of the catalyst in the same described apparatus as above was contacted with 1:1 $H_2$/CO, at a bed temperature of 250° to 270°C, a standard hourly space velocity (SHSV) of 1000 v/v/hr. at 300 psig (2068.5 kPa gauge), for 12 hours. The products were collected and the product distribution data were analyzed by gas chromatography. Results are given below in Table IV.

TABLE IV

| | |
|---|---|
| % CO Conversion | 98 |
| % CO to $CO_2$ | 43 |
| % CO to HC | 55 |
| Wt. % Selectivity $CH_4$ | 7.2 |
| $C_2$=/$C_2$° | 2.6 |
| $C_2$/$C_1$ | 2.1 |
| % $C_2$–$C_6$ | 50.8 |
| % Olefins (of $C_2$–$C_6$ total) | 86 |
| $C_7^+$ | 42 |

As is seen from the data, the Fe–Co Catalyst B generates a $C_2$–$C_6$ fraction which is olefin rich even at high conversion conditions.

EXAMPLE 5

Catalyst B and the control, prepared by the procedure described in Example 1, were pretreated by the procedure described in Example 3 in the apparatus described in Example 2.

Each catalyst in 8 cm³ volume, after pretreatment, was contacted with 1:1 $H_2$/CO at 300 psig (2068.5 kPa gauge) pressure, 1000 v/v/hr. (SHSV) for 12 hour run times at the temperatures listed below in Table V, in same apparatus described in Example 2. Product samples were collected and analyzed after 12 hours onstream with CO/$H_2$.

TABLE V

| | Catalyst B | Control | Control |
|---|---|---|---|
| % CO Conversion | 98 | 67 | 87 |
| % CO to $CO_2$ | 40 | 31 | 37 |
| % CO to HC | 58 | 36 | 50 |
| Temp. °C | 270 | 305 | 340 |
| $C_2$:$C_1$ | 2.2 | 1.2 | 0.7 |
| % $C_2$–$C_6$ | 62 | 41 | 53 |
| % Olefin (of $C_2$–$C_6$ total) | 89 | 88 | 70 |
| Weight % Selectivity | | | |
| $C_1$ | 7.4 | 5.8 | 19.0 |
| $C_2$° | 4.4 | 1.3 | 7.8 |
| $C_2$= | 11.6 | 5.4 | 5.7 |
| $C_3$° | 1.5 | 1.0 | 2.6 |
| $C_3$= | 20.0 | 9.4 | 15.9 |
| $C_4$° | tr. | 1.4 | 2.0 |
| $C_4$= | 11.3 | 8.8 | 8.6 |
| $C_5$° | 0.3 | 1.0 | 1.1 |
| $C_5$= | 7.4 | 7.0 | 4.0 |
| $C_6$° | 0.8 | 0.3 | 2.6 |
| $C_6$= | 4.6 | 5.0 | 3.0 |
| $C_7^+$ | 30.7 | 53.6 | 27.7 |

As is seen from the data, the catalyst derived from the cobalt containing spinel provided greater activity, i.e. 98% CO conversion, than the all-iron oxide control catalysts even though they were operated at 35°C and 70°C higher temperatures. The Fe–Co catalyst generated more $C_2$–$C_6$ olefins than either of the control catalysts and substantially less methane than the control catalyst at high conversion (about 87%) conditions.

## EXAMPLE 6

### Catalyst Preparation

Following the general procedure described in Example 1 the following catalysts were prepared having the empirical formula: $Fe_{3-y}Co_yO_4/1\%K$: where y = 0.03, 0.15, 0.375 and 0.75, respectively. The surface areas of the obtained materials were in the range of 0.1 to 0.5 $m^2/g$.

The above-prepared catalysts were pretreated by the procedure described in Example 2 and in the apparatus described in Example 4, and subjected to hydrocarbon synthesis under the following reaction conditions:

Temperature = 295 ± 10°C
Pressure (gauge) = 300 psig (2068.5 kPa)
Space Velocity = 1000 v/v/hr.
$H_2/CO$ ratio = 1:1
Run Time = 12 hours
Catalyst = 8 $cm^3$ volume, 20–80 mesh size
Analysis of products were performed after 12 hours of run time. Results are shown in Table VI below.

**TABLE VI**

| Performance of $Fe_{3-y}Co_yO_4/1\%K$ | | | | |
|---|---|---|---|---|
| y = | 0.03 | 0.15 | 0.375 | 0.80 |
| % CO Conversion | 97 | 98 | 97 | 98 |
| To $CO_2$ | 27 | 40 | 41 | 42 |
| To HC's | 70 | 58 | 56 | 56 |
| Wt. % Selectivity | | | | |
| $CH_4$ | 8.3 | 7.4 | 18.0 | 13.2 |
| $C_2^=-C_6^=$ | 46.5 | 53.1 | 41.4 | 53.0 |
| $C_2^°-C_6^°$ | 6.9 | 7.2 | 13.3 | 10.6 |
| $C_{7+}$ | 38.3 | 32.3 | 27.3 | 23.2 |

The results show the importance of maintaining the Fe:Co atomic ratio within the preferred range i.e. y = 0.03 to y = 0.40 at the specific conditions in this Example, excessive levels of $CH_4$ are generated at high cobalt levels, i.e., y = 0.375 where Fe:Co = 7:1.

## EXAMPLE 7

This example shows the performance of Catalyst C, $Fe_{2.625}Co_{0.375}O_4$ in hydrocarbon synthesis at different temperatures.

The catalyst was pretreated according to the procedure described in Example 2 and in the same described apparatus. The hydrocarbon synthesis runs were conducted at the indicated temperatures using 8 $cm^3$ volume of catalyst being undiluted with quartz and 20–80 mesh particle size at 1:1 $H_2/CO$, 1000 v/v/hr. (SHSV), 300 psig (2068.5 kPa gauge) for 1–12 hours onstream.

**TABLE VII**

| Performance of $Fe_{2.625}Co_{0.375}O_4/1\%K$ | | | | | | |
|---|---|---|---|---|---|---|
| Furnace Temp. °C | 225 | 240 | 260 | 270 | 280 | 290 |
| Bed Temp. °C | 230 | 248 | 304 | 325 | 331 | 340 |
| % CO Conversion | 30 | 31 | 97 | 98 | 98 | 98 |
| To $CO_2$ | 4 | 7 | 40 | 33 | 41 | 41 |
| To HC's | 26 | 24 | 57 | 55 | 57 | 57 |
| Wt. % Selectivity – $CO_2$-free basis | | | | | | |
| $CH_4$ | 8.1 | 8.2 | 19.1 | 16.7 | 18.3 | 19.1 |
| $C_2^=-C_5^=$ | 42.3 | 55.3 | 37.1 | 31.9 | 37.8 | 24.8 |
| $C_2^°-C_5^°$ | 14.4 | 22.0 | 17.7 | 10.6 | 13.2 | 14.8 |
| $C_6^+$ | 35.2 | 34.5 | 26.1 | 40.8 | 30.7 | 41.3 |

As seen from the data, the change in $CH_4$ selectivity as a function of temperature-conversion indicates that catalysts which contain relatively high levels of cobalt, i.e. an iron/cobalt atomic ratio of 7.0, while useful should be operated at lower temperature-conversion conditions to achieve low $CH_4$ productivity. As further seen in the data, good $C_2$–$C_6$ olefin selectivity is achieved over the entire operating range. The system provided optimal performance in runs where the bed temperature was lower than 304°C.

## EXAMPLE 8

This example shows the improved performance of Catalyst C, $Fe_{2.625}Co_{0.375}O_4$, at low (150 psig, 1034.3 kPa) gauge pressure relative to (300 psig, 2068.5 kPa) high pressure conditions. The catalyst was prepared by the procedure outlined in Example 1, and subjected to the pretreatment and operating procedures substantially as described in Examples 2 and 4, respectively.

The results in Table VIII below show that even at relatively high cobalt levels, i.e. Fe:Co of 7.0, good olefin selectivity and high conversion can be achieved at lower pressures, i.e. 150 psig (1034.3 kPa gauge).

### TABLE VIII

Performance of $Fe_{2.625}Co_{0.375}O_4/1\%K$
at 150 and 300 psig (1034 and 2068 kPa gauge)

| Pressure (psig) kPa gauge | (150) 1034.3 | (300) 2068.5 |
|---|---|---|
| % CO Conversion | 92 | 97 |
| % To $CO_2$ | 38 | 41 |
| % To HC | 54 | 56 |
| Wt. % Selectivity ($CO_2$-free basis) | | |
| $CH_4$ | 7.2 | 17.9 |
| $C_2^=$–$C_5^=$ | 53.4 | 38.1 |
| $C_2^\circ$–$C_5^\circ$ | 4.5 | 12.7 |
| $C_6^+$ | 34.9 | 31.3 |

## EXAMPLE 9

This example shows the effect of $H_2$ treatment at 350°C to reduce $CH_4$ selectivity of an "aged catalyst", in this case Catalyst B, which had been onstream for 72 hours. It is believed that the treatment with $H_2$ at 350°C for 5 hrs. at 100 psig (689.5 kPa gauge), 750 SHSV, removes a carbonaceous surface layer which develops on the catalyst during extended operating periods. The procedures described in Examples 1, 3 and 3 were used to respectively prepare, pretreat, and operate this catalyst under the hydrocarbon synthesis conditions of 270°C, 0.66:1 $H_2/CO$, 2000 v/v/hr. (SHSV), 300 psig (2068 kPa gauge), 50% catalyst dilution with quartz powder in 8 $cm^3$ total volume, catalyst particle size of 20–80 mesh.

### TABLE IX

$H_2$ Treatment Improves Time Dependent
Performance of $Fe_{2.85}Co_{.15}O_4/1\%K$

| Hours on stream | 72[a] | 96[b] |
|---|---|---|
| % CO Conversion | 48 | 62 |
| % CO to $CO_2$ | 23 | 28 |
| % CO to HC | 25 | 34 |
| Wt. % Selectivity ($CO_2$-free basis) | | |
| $CH_4$ | 12.0 | 7.9 |
| $C_2^=$–$C_5^=$ | 43.3 | 46.3 |
| $C_2^\circ$–$C_5^\circ$ | 7.1 | 6.6 |
| $C_6^+$ | 37.6 | 40.1 |

[a]Prior to hydrogen rejuvenation.
[b]After 72 hours on stream, $H_2$ treatment described above, then additional 24 hours on stream with $CO/H_2$.

EXAMPLE 10

This example demonstrates the performance of Catalyst B, $Fe_{2.85}Co_{0.15}O_4$, at various temperatures under hydrocarbon synthesis conditions. The catalyst was 50% diluted with quartz powder as described in the previous Example. The respective procedures outlined in Examples 1 and 3 were used to prepare, pretreat and operate this catalyst under the hydrocarbon synthesis conditions listed below in Table X.

TABLE X

$Fe_{2.85}Co_{.15}O_4/1\%K$ Performance

| Run | 1 | | 2 | | 3 | |
|---|---|---|---|---|---|---|
| Temp. °C | 230 | | 250 | | 270 | |
| Gauge Pressure (psig) kPa | (300) | 2068.5 | (300) | 2068.5 | (300) | 2068.5 |
| $H_2/CO$ | 1.0 | | 1.0 | | 1.0 | |
| SHSV | 1800 | | 1800 | | 1800 | |
| | | | | | | |
| % CO Conv. | 36.4 | | 97.5 | | 98.4 | |
| HR on stream | 2 | | 4 | | 6 | |
| % CO to $CO_2$ | 14 | | 44.0 | | 43.0 | |
| % CO to HC | 22.4 | | 53.5 | | 55.4 | |
| Wt. % Selectivity ($CO_2$-free basis) | | | | | | |
| $CO_2$ | 37.9 | | 45.0 | | 43.8 | |
| $CH_4$ | 1.3 | (2.1) | 2.6 | (4.7) | 3.2 | (5.7) |
| $C_2^-$ | 2.0 | (3.22) | 3.0 | (5.5) | 3.4 | (6.0) |
| $C_2^\circ$ | 0.4 | (0.6) | 0.8 | (1.5) | 0.8 | (1.4) |
| $C_3^-$ | 4.1 | (6.6) | 5.4 | (9.8) | 6.3 | (11.2) |
| $C_3^\circ$ | 0.8 | (1.3) | 0.6 | (1.1) | 0.6 | (1.1) |
| $C_4^-$ | 1.7 | (2.7) | 3.4 | (6.2) | 4.0 | (7.1) |
| $C_4^\circ$ | 0.1 | (0.2) | 0.5 | (0.9) | 0.5 | (0.9) |
| $C_5^-$ | 1.4 | (2.3) | 2.6 | (4.7) | 3.5 | (6.2) |
| $C_5^\circ$ | 0.3 | (0.5) | 0.5 | (0.9) | 0.9 | (1.6) |
| $C_6^-$ | 1.2 | (1.9) | 1.9 | (3.5) | 2.2 | (3.9) |
| $C_6^\circ$ | 0.4 | (0.6) | 0.3 | (0.5) | 0.3 | (0.5) |
| $C_7^+$ | 48.4 | (78.0) | 33.4 | (60.7) | 30.5 | (54.4) |

EXAMPLE 11

This example demonstrates the performance of Catalyst B, $Fe_{2.85}Co_{0.15}O_4$ at various temperatures in the form of undiluted catalyst. The catalyst was prepared by the procedure described in Example 1 and pretreated and operated as respectively described in Examples 2 and 4. The process conditions for each run are listed below in Table XI. In contrast to Run 4 shown below, bed dilution as employed in Example 10 allows the system to operate under more isothermal conditions thereby minimizing the extent of carbon and carbonaceous deposit formation.

TABLE XI

Fe$_{2.85}$Co$_{.15}$O$_4$/1%K Performance
Undiluted Bed

| Run | 1 | 2 | 3 | 4 |
|---|---|---|---|---|
| SHSV: | 1000 | 1000 | 570 | 570 |
| Temp. | 235 | 270 | 235 | 270 |
| H$_2$:CO | 1.0 | 1.0 | 1.0 | 1.0 |
| Press (psig) kPa gauge | (300) 2068.5 | (300) 2068.5 | (300) 2068.5 | (300) 2068.5 |
| Time on stream hr. | 8 | 10 | 16 | 18 |
| % CO Conv. | 29.4 | 98.0 | 49.1 | 98.0* |
| % CO to CO$_2$ | 8.0 | 42.0 | 22.0 | 40.0 |
| % CO to HC | 21.4 | 56.0 | 27.1 | 58.0 |
| Wt. % Select. (Co$_2$-free basis) | | | | |
| CO$_2$ | 26.2 | 42.5 | 43.5 | 40.2 |
| CH$_4$ | 1.9 (2.6) | 3.0 (5.2) | 1.7 (2.0) | 3.7 (6.2) |
| C$_2$$^=$ | 4.3 (5.8) | 4.5 (7.8) | 2.5 (4.4) | 4.0 (6.7) |
| C$_2$° | 1.4 (1.9) | 1.7 (2.9) | 0.9 (1.6) | 1.7 (2.8) |
| C$_3$$^=$ | 6.4 (8.7) | 7.8 (13.4) | 6.6 (11.6) | 8.3 (13.8) |
| C$_3$° | 0.6 (0.8) | 0.6 (1.0) | 0.7 (1.2) | 0.8 (1.3) |
| C$_4$$^=$ | 1.4 (1.9) | 4.4 (7.6) | 2.5 (4.4) | 3.8 (6.3) |
| C$_4$° | tr. (tr.) | 0.2 (0.3) | 0.4 (0.7) | 3.5 (0.8) |
| C$_5$$^=$ | 0.9 (1.2) | 2.8 (4.8) | 1.7 (2.9) | 2.7 (4.5) |
| C$_5$° | tr. (tr.) | 0.1 (0.2) | 0.4 (0.7) | 0.35 (0.8) |
| C$_6$$^+$ | 56.9 (76.8) | 32.4 (55.9) | 39.1 (68.6) | 34.2 (57.0) |

*Note: Bed plugging with wax and carbonaceous deposits limited continuous operating periods to ≤ 40–50 hrs.

## Claims

1. A process for synthesizing a hydrocarbon mixture containing C$_2$ to C$_6$ olefins from a feed gas containing CO and H$_2$ comprising passing the feed gas in contact with a carbided and reduced, unsupported iron-cobalt single phase spinel catalyst, said spinel having the initial empirical formula:

Fe$_x$Co$_y$O$_4$

wherein x and y are integer or decimal values, other than zero, with the provisos that the sum of x + y is 3 and the ratio of x/y is 7:1 or above, said spinel exhibiting a single phase X-ray diffraction pattern substantially isostructural with that of Fe$_3$O$_4$ at a temperature in the range of from 200 to 300°C, a (gauge) pressure in the range of from 50 to 100 psig (344.8 to 6895 kPa) and for a sufficient time to form a hydrocarbon mixture containing C$_2$ to C$_6$ olefins, characterized in that the said spinel has a measured B.E.T. surface area in the range of from 0.1 to 5 m²/g.

2. A process according to claim 1 in which said catalyst further comprises a promoter agent present in an amount of from 0.1 to 10 gram-atom percent of the total gram-atoms of metal content.

3. A process according to claim 2 in which the said promoter agent is selected from bicarbonates, carbonates, organic acid salts, inorganic acid salts, nitrates, sulfates, halides and hydroxides of Group IA and IIA metals (e.g. potassium carbonate).

4. A process according to any one of claims 1 to 3 further characterized in that the said spinel is of the formula: $Fe_{2.85}Co_{0.15}O_4$, $Fe_{2.625}Co_{0.375}O_4$ or $Fe_{2.97}Co_{0.03}O_4$, or comprises an unsupported iron-cobalt spinel composition of the formula: $Fe_{2.85}Co_{0.15}O_4/1\%K$.

5. A process according to any one of claims 1 to 4 further characterized in that the said contacting of said catalyst and said CO and hydrogen is carried out in either a fixed bed reactor system or a slurry phase reactor system.

6. A process according to claim 5 further characterized in that said slurry phase system is comprised of an admixture of said catalyst which a slurry liquid selected from high boiling liquid paraffins, aromatic hydrocarbons, ethers, amines, or mixtures thereof.

7. A process according to claim 6 further characterized in that the said high boiling liquid paraffins are $C_{12}–C_{60}$ linear or branched saturated aliphatic hydrocarbons.

8. A process according to any one of claims 5 to 7 in which in the slurry phase reactor system, the weight ratio of slurry liquid:catalyst taken as the dry basis, is the range of 10:1 to 5:1.

9. A process according to claim 5 performed in a fixed bed reactor system and in which the said catalyst is admixed with a solid diluent.

10. A process according to any one of claims 5 to 9 comprising the step of recovering a product hydrocarbon mixture containing from 25 to 80 wt.% of $C_2$ to $C_6$ olefins, based on the total weight of hydrocarbons produced.

**Patentansprüche**

1. Verfahren zum Synthetisieren einer $C_2–C_6$-Olefine enthaltenden Kohlenwasserstoffmischung aus einem CO und $H_2$ enthaltenden Einsatzmaterialgas, bei dem das Einsatzmaterialgas im Kontakt mit einem einer Karbidbildungsbehandlung unterworfenen und reduzierten, nicht auf einem Träger aufgebrachten Eisen-Kobalt-Einphasenspinellkatalysator, der die anfängliche empirische Formel:

$Fe_xCo_yO_4$

besitzt, in der x und y von 0 abweichende ganze Zahlen oder Dezimalwerte mit der Maßgabe sind, daß die Summe von x + y 3 ist und das Verhältnis von x/y 7:1 oder größer ist, wobei der Spinell ein Einphasen-Röntgenbeugungsspektrum zeigt, das im wesentlichen isostrukturell mit dem von $Fe_3O_4$ ist, bei einer Temperatur im Bereich von 200 bis 300°C, einem Überdruck im Bereich von 344,8 bis 6895 kPa und über einen für die Bildung einer $C_2–C_6$-Olefine enthaltenden Kohlenwasserstoffmischung ausreichend langen Zeitraum geführt wird, dadurch gekennzeichnet, daß der Spinell eine gemessene B.E.T.-Oberfläche im Bereich von 0,1 bis 5 $m^2/g$ aufweist.

2. Verfahren nach Anspruch 1, bei dem der Katalysator außerdem ein Promotormittel enthält, das, bezogen auf den Gesamtmetallgehalt in Grammatomen, in einer Menge von 0,1 bis 10 Grammatomprozent vorhanden ist.

3. Verfahren nach Anspruch 2, bei dem das Promotormittel ausgewählt ist aus Bicarbonaten, Carbonaten, Salzen organischer Säuren, Salzen anorganischer Säuren, Nitraten, Sulfaten, Halogeniden und Hydroxiden von Gruppe IA und IIA Metallen (z.B. Kaliumcarbonat).

4. Verfahren nach einem der Ansprüche 1 bis 3, ferner dadurch gekennzeichnet, daß der Spinell die Formel $Fe_{2.85}Co_{0.15}O_4$, $Fe_{2.625}Co_{0.375}O_4$ oder $Fe_{2.97}Co_{0.03}O_4$ besitzt oder eine nicht auf einen Träger aufgebrachte Eisen-Kobalt-Spinellzusammensetzung mit der Formel $Fe_{2.85}Co_{0.15}O_4/1\%$ K umfaßt.

5. Verfahren nach einem der Ansprüche 1 bis 4, weiterhin dadurch gekennzeichnet, daß das Kontaktieren des Katalysators und des CO und Wasserstoffs entweder in einem Festbettreaktorsystem oder in einem Aufschlämmungsphasereaktorsystem durchgeführt wird.

6. Verfahren nach Anspruch 5, weiterhin dadurch gekennzeichnet, daß das Aufschlämmungsphasesystem aus einem Gemisch des Katalysators mit einer Aufschlämmungsflüssigkeit ausgewählt aus hochsiedenden flüssigen Paraffinen, aromatischen Kohlenwasserstoffen, Ethern, Aminen oder Mischungen derselben zusammengesetzt ist.

7. Verfahren nach Anspruch 6, weiterhin dadurch gekennzeichnet, daß die hochsiedenden flüssigen Paraffine lineare oder verzweigte gesättigte aliphatische $C_{12}–C_{60}$-Kohlenwasserstoffe sind.

8. Verfahren nach einem der Ansprüche 5 bis 7, bei dem das Gewichtsverhältnis von Aufschlämmungsflüssigkeit zu Katalysator auf Trockenbasis im Aufschlämmungsphasereaktorsystem im Bereich von 10:1 bis 5:1 liegt.

9. Verfahren nach Anspruch 5, durchgeführt in einem Festbettreaktor, bei dem der Katalysator mit einem festen Verdünnungsmittel vermischt ist.

10. Verfahren nach einem der Ansprüche 5 bis 9, das die Stufe der Gewinnung einer Produktkohlenwasserstoffmischung umfaßt, die bezogen auf das Gesamtgewicht der hergestellten Kohlenwasserstoffe 25 bis 80 Gew.% $C_2–C_6$-Olefine enthält.

**Revendications**

1. Un procédé pour synthétiser un mélange d'hydrocarbures contenant des oléfines en $C_2$ à $C_6$ à partir d'une charge de gaz renfermant CO et $H_2$, consistant à faire passer la charge de gaz en contact avec un catalyseur à spinelle mono-phase à fer-cobalt non-supporté, carburé et réduit, ledit spinelle présentant la formule initiale empirique

$Fe_xCo_yO_4$

dans laquelle x et y sont des valeurs entières ou décimales, autres que zéro, pourvu que la somme x + y est 3 et le rapport x/y est 7:1 ou au dessus, ledit spinelle présentant un diagramme de diffraction mono-phase aux rayons X, sensiblement isostructurel à celui de $Fe_3O_4$ à une température de 200 à 300°C, une pression de 50 à 1000 psig (344,8 à 6895 k Pa) et pendant un temps suffisant pour former un mélange d'hydrocarbures renfermant des oléfines en $C_2$ à $C_6$, caractérisé en ce que ledit spinelle présente une surface spécifique B.E.T. mesurée de 0,1 à 5 m2/g.

2. Un procédé selon la revendication 1, dans lequel ledit catalyseur comprend en outre un agent d'activation présent en une quantité de 0,1 à 10 pourcent d'atome-grammes par rapport au total d'atome-grammes de la teneur en métal.

3. Un procédé selon la revendication 2, dans lequel ledit agent d'activation est choisi parmi les bicarbonates, les carbonates, les sels d'acide organique, les sels d'acide minéraux, les nitrates, les sulfates, les halogénures et les hydroxydes des métaux des Groupes IA et IIA (par exemple le carbonate de potassium).

4. Un procédé selon l'une quelconque des revendications 1 à 3, caractérisé en outre en ce que ledit spinelle présente la formule: $Fe_{2,85}Co_{0,15}O_4$, $Fe_{2,625}Co_{0,375}O_4$ ou $Fe_{2,97}Co_{0,03}O_4$, ou comprend une composition à spinelle au fer-cobalt, non supportée de formule: $Fe_{2,85}Co_{0,15}O_4/1\%K$.

5. Un procédé selon l'une quelconque des revendications 1 à 4, caractérisé en outre en ce que ladite mise en contact du CO et de l'hydrogène avec ledit catalyseur est réalisée soit dans un système de réacteur à lit fixe, soit dans un système de réacteur à phase en suspension épaisse.

6. Un procédé selon la revendication 5, caractérisé en outre en ce que le système de réacteur à suspension épaisse est constitué d'un mélange dudit catalyseur avec un liquide en suspension épaisse choisis parmi les paraffines liquides, les hydrocarbures aromatiques, les éthers, les amines à point d'ébullition élevé ou leurs mélanges.

7. Un procédé selon la revendication 6, caractérisé en outre en ce que lesdites paraffines liquides à point d'ébullition élevé sont des hydrocarbures diphatiques saturés en $C_{12}$ à $C_{60}$, linéaires ou ramifiés.

8. Un procédé selon l'une quelconque des revendications 5 à 7, dans lequel, dans le système de réacteur à phase en suspension épaisse, le rapport pondéral de la suspension liquide au catalyseur, pris à l'état sec, est dans la plage de 10:1 à 5:1.

9. Un procédé selon la revendication 5, mis en œuvre dans un système de réacteur à lit fixe et dans lequel ledit catalyseur est mélangé avec un diluant solide.

10. Un procédé selon l'une quelconque des revendications 5 à 9, comprenant l'étape de récupération d'un produit mélange d'hydrocarbures renfermant de 25 à 80% en poids d'oléfine en $C_2$ à $C_6$, en se basant sur le poids total des hydrocarbures produits.